Europäisches Patentamt

European Patent Office

Office européen des brevets

(11) Numéro de publication: **0 305 277**
**A2**

# DEMANDE DE BREVET EUROPEEN

(21) Numéro de dépôt: **88402122.1**

(51) Int. Cl.⁴: **A 61 K 31/425**

(22) Date de dépôt: **18.08.88**

(30) Priorité: **25.08.87 FR 8711884**

(43) Date de publication de la demande:
**01.03.89 Bulletin 89/09**

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony (FR)**

(72) Inventeur: **Blanchard, Jean-Charles**
**107 Avenue de Paris**
**F-94160 Saint Mande (FR)**

**Laduron, Pierre**
**5 Rue des Wallons**
**F-75013 Paris (FR)**

**Stutzmann, Jean-Marie**
**9 Rue de l'Arche**
**F-94440 Villecresnes (FR)**

(74) Mandataire: **Savina, Jacques et al**
**RHONE-POULENC INTERSERVICES Service Brevets**
**Pharma 25, quai Paul Doumer**
**F-92408 Courbevoie Cédex (FR)**

(54) Application de l'amino-2 trifluoro-méthoxy-6 benzothiazole pour obtenir un médicament destiné au traitement des troubles du sommeil et de la dépression.

(57) Application de l'amino-2 trifluorométhoxy-6 benzothiazole pour obtenir un médicament régulateur du sommeil, utile dans le traitement des troubles du sommeil et dans le traitement de la dépression.

EP 0 305 277 A2

**Description**

## APPLICATION DE L'AMINO-2 TRIFLUOROMETHOXY-6 BENZOTHIAZOLE POUR OBTENIR UN MEDICAMENT DESTINE AU TRAITEMENT DES TROUBLES DU SOMMEIL ET DE LA DEPRESSION

La présente invention concerne l'application de l'amino-2 trifluorométhoxy-6 benzothiazole ou un sel pharmaceutiquement acceptable de ce composé pour obtenir un médicament régulateur du sommeil utile dans le traitement des troubles du sommeil et dans le traitement de la dépression.

Du brevet européen 50 551, il est connu que l'amino-2 trifluorométhoxy-6 benzothiazole ou un sel pharmaceutiquement acceptable de ce composé est utile comme médicament anticonvulsivant, anxiolytique et hypnotique.

Il a maintenant été trouvé que l'amino-2 trifluorométhoxy-6 benzothiazole ou un sel pharmaceutiquement acceptable de ce composé agit sur le sommeil à ondes lentes mais également sur le sommeil paradoxal. Ce composé est donc utile dans le traitement des troubles du sommeil et le traitement de la dépression, maladie dont on sait que la quantité de sommeil paradoxal et la latence d'apparition des phases de sommeil paradoxale sont diminuées (MENDLEWICZ et al, Acta Psychiat. Scand. 320, 26-29, 1985).

Les propriétés sur le sommeil ont été déterminées chez le rat selon le protocole suivant :

Chez des rats mâles (CD, C.O.B.S., Ch. RIVER, FRANCE), de 300 à 400 g, des électrodes corticales (aires somesthésiques et visuelles) et musculaires (muscle de la nuque) sont implantées à demeure. Une semaine plus tard, les enregistrements polygraphiques peuvent commencer. D'un point de vue électroencéphalographique (EEG), on définit 4 stades correspondant à différents niveaux de vigilance chez le rat :
- éveil
- somnolence
- sommeil à ondes lentes
- sommeil paradoxal.

Pour chaque rat, on recueille les tracés EEG pendant 7 heures la veille de l'administration (jour $J_1$) et le jour de l'administration (jour $J_2$) du produit. On calcule ensuite le pourcentage de répartition des différents stades du cycle veille-sommeil par rapport à la durée totale de l'enregistrement. Pour chaque dose de produit, on compare alors les pourcentages moyens calculés pour chacun des stades le jour $J_1$ avec les pourcentages moyens correspondants calculés le jour $J_2$.

Le composé est administré par voie orale (5 ml/kg) aux doses de 0,5, 2 et 8 mg/kg en solution dans 0,2 ml d'acide chlorhydrique 1N. On utilise 4 à 5 animaux par dose.

A la dose de 0,5 mg/kg p.o., le composé ne modifie pas la répartition du cycle veille-sommeil. Par contre, aux doses de 2 et 8 mg/kg p.o., ce composé diminue significativement l'éveil (respectivement -27 %, $p < 0,01$ et -39 %, $p < 0,01$) au profit du sommeil à ondes lentes (respectivement + 46 %, $p < 0,05$ et + 57 %, $p < 0,05$) et du sommeil paradoxal (respectivement + 84 %, $p < 0,01$ et + 79 %, $p < 0,05$).

Le composé augmente donc globalement le sommeil à ondes lentes et le sommeil paradoxal au détriment de l'éveil dès la dose de 2 mg/kg p.o. chez le rat.

L'amino-2 trifluorométhoxy-6 benzothiazole présente une faible toxicité. Sa $DL_{50}$ par voie orale chez la souris est de 67 mg/kg. Cette $DL_{50}$ a été calculée après 3 jours d'observation par la méthode cumulative de J.J. REED et H. MUENCH. Amer J. HYG 1938, 27, 493.

L'amino-2 trifluorométhoxy-6 benzothiazole peut être préparé selon le procédé décrit dans le brevet EP 50 551.

Les médicaments régulateurs du sommeil sont constitués par l'amino-2 trifluorométhoxy-6 benzothiazole ou un sel pharmaceutiquement acceptable de ce composé à l'état pur ou sous forme d'une composition dans laquelle il est associé à tout autre produit pharmaceutiquement compatible, pouvant être inerte ou physiologiquement actif. Ces médicaments peuvent être utilisée par voie orale, parentérale ou rectale.

Comme compositions solides pour administration orale peuvent être utilisés des comprimés, pilules, poudres (capsules de gélatine, cachets) ou granulés. Dans ces compositions, le principe actif est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'ethanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, l'amino-2 trifluorométhoxy-6 benzothiazole ou un sel pharmaceutiquement acceptable de ce composé est utile comme régulateur du sommeil notamment dans le traitement des troubles du sommeil et dans le traitement de la dépression.

Les doses dépendent de l'effet recherché, de la durée du traitement et de la voie d'administration utilisée ; elles sont généralement comprises entre 10 et 100 mg par jour par voie orale pour un adulte avec des doses unitaires allant de 2 à 50 mg de substance active.

D'une façon générale, le médecin déterminera la posologie appropriée en fonction de l'âge, du poids et de tous les autres facteurs propres au sujet à traiter.

Les exemples suivants illustrent des compositions pharmaceutiques utilisables comme régulateur du sommeil.

Exemple A

On prépare, selon la technique habituelle, des gélules dosées à 25 mg de produit actif ayant la composition suivante :
- amino-2 trifluorométhoxy-6 benzothiazole   25 mg
- cellulose microcristalline   75 mg
- mannitol   41 mg
- silice colloïdale   4 mg
- carboxyméthylamidon sodique   25 mg
- talc   18 mg
- stéarate de magnésium   2 mg
- polyvidone excipient   10 mg

Exemple B

On prépare, selon la technique habituelle, des comprimés dosés à 50 mg de produit actif ayant la composition suivante :
- amino-2 trifluorométhoxy-6 benzothiazole   50 mg
- cellulose monocristalline   75 mg
- mannitol   41 mg
- polyvidone excipient   10 mg
- carboxyméthylamidon   25 mg
- silice colloïdale   4 mg
- talc   18 mg
- stéarate de magnésium   2 mg
- mélange d'hydroxyméthylcellulose, glycérine, oxyde de titane (72-3,5-24,5)   q.s.p. 1 comprimé pelliculé terminé à 245 mg

Exemple C

On prépare une solution injectable contenant 10 mg de produit actif ayant la composition suivante :
- amino-2 trifluorométhoxy-6 benzothiazole   10 mg
- acide benzoïque   80 mg
- alcool benzylique   0,06 cm³
- benzoate de sodium   80 mg
- éthanol à 95 %   0,4 cm³
- hydroxyde de sodium   24 mg
- propylèneglycol   1,6 cm³
- eau   q.s.p. 4 cm³

**Revendications**

1 - Application de l'amino-2 trifluorométhoxy-6 benzothiazole ou un sel pharmaceutiquement acceptable de ce composé pour obtenir un médicament utile comme régulateur du sommeil.

2 - Application selon la revendication 1 caractérisée en ce que le médicament est destiné au traitement de la dépression.

3 - Application selon la revendication 1 caractérisée en ce que le médicament est destiné au traitement des troubles du sommeil.